# EUROPÄISCHE PATENTSCHRIFT

(11) **EP 1 049 430 B1**
(45) Veröffentlichungstag und Bekanntmachung des Hinweises auf die Patenterteilung: **07.07.2004**
(21) Anmeldenummer: 99901495.4
(22) Anmeldetag: 22.01.1999
(51) Int. Cl.: A61F 2/44

(54) **IMPLANTAT**
IMPLANT
IMPLANT

(30) Priorität: 23.01.1998 AT 11098
(43) Veröffentlichungstag der Anmeldung: 08.11.2000
(73) Patentinhaber: Aesculap AG & Co. KG, 78532 Tuttlingen (DE)
(72) Erfinder: Fuss, FranzK., 2700 Wiener Neustadt (AT); Sabitzer, Ronald, J., 1160 WIEN (AT)
(74) Vertreter: Böhme, Ulrich, Dr. Dipl.-Phys.
(86) Internationale Anmeldenummer: PCT/AT1999/000015
(87) Internationale Veröffentlichungsnummer: WO 1999/037255

(56) Entgegenhaltungen:
- EP-A- 0 599 419
- WO-A-96/40014
- WO-A-97/23175
- FR-A- 2 736 537

## Beschreibung

Die vorliegende Erfindung bezieht sich auf ein Implantat zum Einsetzen zwischen Wirbelkörper der Wirbelsäule, wobei das im Querschnitt im wesentlichen rechteckige oder trapezoidförmige Implantat an zu benachbarten Wirbelkörpern gewandten Deckflächen porös und/oder mit einer Profilierung ausgebildet ist, mit einer Breite, welche maximal die zum Dornfortsatz gewandte, rückwärtige Hälfte des Wirbelkörpers überdeckt, und mit einer den rückwärtigen Bereich des Wirbelkörpers im wesentlichen übergreifende Länge, wobei das Implantat an seiner zum Inneren des Wirbelkörpers gewandten, in Richtung benachbarter Wirbelkörper verlaufenden Begrenzungsfläche konvex gekrümmt ist und an seiner zum Dornfortsatz gewandten Begrenzungsfläche konkav gekrümmt ausgebildet ist, so daß sich eine sichelförmige Ausgestaltung ergibt.

Derartige Implantate dienen zur Ausbildung von Stützen für Wirbelkörperfusionen, wobei nach wenigstens teilweisem Ausräumen einer Bandscheibe und Vorbereitung der Deckplatten der Wirbelkörper das zwischen benachbarte Wirbelkörper einzusetzende Implantat in den Intervertebrafraum eingebracht wird und den Zwischenwirbelraum sicher freihält. Durch die Ausbildung der zu benachbarten Wirbelkörpem gewandten Deckflächen mit einer porösen bzw. mit einer mit einer Profilierung versehenen Oberfläche kann nach dem Einsetzen des Implantates eine sichere Verankerung durch Einwachsen der Knochen der benachbarten Wirbelkörper an den Deckflächen des Implantates erzielt werden. Alternativ oder zusätzlich kann wenigstens eine Durchbrechung bzw. Ausnehmung an der Deckfläche eines derartigen Implantates vor dem Einsetzen mit Knochenmasse verfüllt werden, worauf nach Einsetzen des Implantates ein Verwachsen der in dem Implantat aufgenommenen Knochenmasse mit dem Material der unmittelbar benachbarten Wirbelkörper angestrebt wird. Zusätzlich kann neben dem Implantat zur Abstützung ein sich über mehrere Wirbelkörper erstreckendes Stützsystems außerhalb der Wirbelsäule vorgesehen sein, welches jeweils an einzelnen Wirbelkörpern festlegbar ist.

Implantate der eingangs genannten Art sind beispielsweise der US-A 4 834 757, US-A 5 192 327 oder der WO 90/00037 zu entnehmen. Bei der Ausbildung gemäß der US-A 4 834 757 finden im wesentlichen eine rechteckigen Querschnitt aufweisende Implantate Verwendung, wobei posterior zwischen jeweils zwei benachbarten Wirbelkörpern jeweils in beiden seitlichen Bereichen der Wirbelkörper zwei Implantate eingesetzt werden. Nachteilig bei dieser bekannten Ausbildung ist neben der für das Einsetzen der Implantate erforderlichen wenigstens teilweisen Entfernung der hinteren knöchernen und ligamentären Elemente die zwingende Notwendigkeit der Verwendung von zwei getrennten Implantaten, weiche nicht nur einen erhöhten operativen Aufwand sondern auch eine nicht ohne weiteres durchführbare gegenseitige korrekte Positionierung erfordern.

Bei der Ausbildung gemäß der WO 97/00037 wird ein Implantat eingesetzt, dessen Außenabmessungen im wesentlichen den Gesamtabmessungen zweier benachbarter Wirbelkörper entsprechen. Bei derartigen Implantaten ist insbesondere nachteilig, daß sie praktisch nur von anterior eingesetzt werden können, wobei jedoch neben einem komplizierten Zugang durch den Bauchraum und einer gegebenenfalls möglichen Verletzung großer Bauchgefäße und des Nervengeflechts ein vor den Wirbelkörpern liegendes, vorderes Längsband durchtrennt werden muß, so daß die natürliche Stützfunktion dieses Längsbandes nach dem Einsetzen des Implantates nicht mehr vorhanden ist. Weiters muß für das somit erforderliche Einsetzen eines zusätzlichen Stützgerüstes eine weitere Operation von posterior vorgenommen werden.

Neben diesen Ausführungsformen für Implantate zum Einsetzen zwischen Wirbelkörpern der obigen Art sind weiters Implantate mit im wesentlichen zylinderförmigen Konturen bekannt, wobei beispielsweise auf die EP-A 0 369 603 oder die DE-C 36 37 314 verwiesen werden kann. Derartige zylinderförmige bzw. rohrförmige Implantate können zusätzlich mit einer gewindeähnlichen Außenkontur versehen sein, um gegebenenfalls durch ein selbsttätiges Einschneiden ein Einschrauben zwischen die Wirbelkörper zu ermöglichen. Nachteilig bei derartigen rohrförmigen Implantaten, bei welchen im Normalfall wiederum jeweils zwei Implantate zwischen jeweils zwei benachbarten Wirbelkörpern verwendet werden müssen, ist insbesondere, daß eine definierte, im wesentlichen ebene Abstützfläche an die Wirbelkörper durch die Implantate nicht erzielbar ist und daß derart gegebenenfalls Schwierigkeiten beim Einwachsen des aufgenommen Knochenmaterials zu befürchten sind. Weiters ist nachteilig, daß durch das Einschneiden bzw. Einschrauben des rohrförmigen Implantates eine Verletzung weicher Knochenteile mit einem damit verbundenen Einbrechen bzw. Einsinken des Knochens hervorgerufen werden kann.

In der WO-A-96/40014 ist ein Wirbelsäulenimplantat beschrieben mit einer konvex gekrümmten Begrenzungsfläche und einer ihr gegenüberliegenden konkav gekrümmten Begrenzungsfläche, welches insgesamt eine etwa sichelförmige Ausgestaltung aufweist. Ausgehend von diesem Stand der Technik liegt der Erfindung die Aufgabe zugrunde, das Implantat so auszubilden, daß das Verwachsen bzw. Anwachsen von Knochenmassen im Zwischenwirbelraum gefördert wird.

Diese Aufgabe wird bei einem Implantat der eingangs beschriebenen Art erfindungsgemäß dadurch gelöst, daß die zum Innern des Wirbelkörpers gewandte Begrenzungsfläche wenigstens eine Vertiefung aufweist.

In eine solche Vertiefung kann Knochenmaterial eingefüllt werden, so daß in dem freibleibenden Bereich des Zwischenwirbelraumes neben dem Implantat Knochenmaterial zur Förderung der Brückenbildung zwischen den benachbarten Wirbeln lokalisiert werden kann, außerdem ergibt sich dadurch eine zuverlässige Lokalisierung und Fixierung des Implantates im Zwischenwirbelraum.

Mit diesem Implantat kann zwischen zwei Wirbelkörpern eine ausreichende und zuverlässige Abstützfunktion erzielt werden, wobei das Implantat von posterior, insbesondere lateral, einsetzbar ist. Dies vereinfacht die Operationstechnik. Das Implantat kann gegebenenfalls gemeinsam mit einem zusätzlich Stützsystem eingesetzt werden. Die spezielle Formgebung sorgt dafür, daß natürlich vorgesehene Stützelemente, wie beispielsweise das vordere Längsband, beim Einsetzen des Implantates nicht beschädigt oder zerstört werden müssen, außerdem ist es nicht notwendig, zusätzlich Bandund Knochenmaterial, insbesondere im Bereich des Wirbel- bzw. Dornfortsatzes, für das Einbringen des Implantates entfernen zu müssen.

Das Implantat hat vorzugsweise eine Breite, welche maximal die zum Dornfortsatz gewandte, rückwärtige Hälfte, vorzugsweise maximal das rückwärtige Drittel, des Wirbelkörpers überdeckt, und eine den rückwärtigen Bereich des Wirbelkörpers im wesentliche übergreifende Länge. Dadurch, daß das Implantat in eingebautem Zustand im wesentlichen lediglich den hinteren Teilbereich benachbarter Wirbelkörper überdeckt, kann mit einem entsprechend kleinbauenden Implantat das Auslangen gefunden werden, wobei in dem hinteren, zum Wirbel- bzw. Dornfortsatz gewandten Teilbereich der Wirbelkörper die größten Kräfte und Beanspruchungen wirksam werden, welche somit entsprechend sicher durch das erfindungsgemäße Implantat aufgenommen werden können, und die Knochenfestigkeit am größten ist.

Neben der kleinbauenden, einteiligen Ausführung des Implantates ist es weiterhin vorteilhaft, daß die konvex gekrümmte Begrenzungsfläche in eingebautem Zustand zum Innern des Wirbelkörpers gewandt sein kann, so daß das Implantat lateral von hinten in den Zwischenwirbelraum zwischen austretender und vorbeiziehender Nervenwurzel und dem Duralsack eingebracht werden kann. Bei einer derartigen posterioren Operationstechnik bleibt nicht nur das vordere Längsband vollkommen unbeschädigt, sondern es kann auch auf das zusätzliche Entfernen von Band- und Knochenmaterial verzichtet werden. Durch die gekrümmte Begrenzungsfläche läßt sich nicht nur ein einfaches seitliches Einbringen des Implantates sicherstellen, sondem es läßt sich auch eine exakte Positionierung des Implantates erzielen, um in eingesetztem Zustand den zum Dornfortsatz gewandten Teilbereich der Wirbelkörper zu überdecken und derart für eine ordnungsgemäße Abstützung zu sorgen.

Durch die zusätzlich konkav gekrümmte Begrenzungsfläche und die im wesentlichen bogen- bzw. sichelförmige Ausgestaltung des Implantates kann dieses leicht und zuverlässig bei äußerst geringem Platzbedarf in den Zwischenwirbetraum von der Seite von hinten eingebracht werden.

Für eine ausreichende Abstützung benachbarter Wirbelkörper durch Überdecken eines entsprechenden Teilbereichs derselben ist es vorteilhaft, wenn der Krümmungsradius der zum Inneren des Wirbelkörpers gewandten Begrenzungsfläche des Implantates 30 bis 90 %, insbesondere 40 bis 80 %, des Abstandes zwischen der Vorderkante des Wirbelkörpers und der Spitze des Dornfortsatzes beträgt.

Für eine Anpassung an die Außenkonturen der Wirbelkörper in dem zum Dornfortsatz gewandten Teilbereich sowie für die Aufrechterhaltung eines ausreichenden Abstandes des Implantates zum Wirbelkanal kann vorgesehen sein, daß der Krümmungsradius der zum Dornfortsatz gewandten Begrenzungsfläche des Implantates 10 bis 80 %, insbesondere 20 bis 60 %, des Abstandes zwischen der Vorderkante des Wirbelkörpers und der Spitze des Dornfortsatzes beträgt. Durch entsprechende Wahl der Krümmungsradien der Begrenzungsflächen läßt sich bei dem Implantat eine Anpassung an unterschiedlich große Wirbelkörper bzw. an Wirbelkörper in unterschiedlichen Bereichen der Wirbelsäule erzielen.

Das Implantat kann bei einer speziellen Ausführungsform vor dem Einsetzen mit Knochenmasse ausgefüllt werden, um in weiterer Folge ein Verwachsen mit umliegendem Knochenmaterial der Wirbelkörper zur Erzielung der gewünschten Abstützung zu ermöglichen. Um ausgehend von dem den rückwärtigen Abschnitt der Wirbelkörper überdeckenden Implantat jedoch eine Ausbildung von Knochenmasse in Richtung zum Dornfortsatz und insbesondere in Richtung zum Wirbelkanal zu vermeiden, ist bevorzugt vorgesehen, daß die zum Dornfortsatz gewandte Begrenzungsfläche im wesentlichen frei von Durchbrechungen ausgebildet ist. Diese Begrenzungsfläche stellt somit eine definierte Außenfläche des Implantates zur Verfügung, bei welcher ein Austritt von Knochenmasse aus dem Inneren des Implantates nicht befürchtet werden muß.

Da das Implantat lediglich einen Teilbereich der Fläche der Wirbelkörper in eingebautem Zustand überdeckt, kann vor dem Einsetzen des Implantates der außerhalb dieses Teilbereichs liegende Zwischenwirbelraum nach Entfernen der Bandscheibe ebenfalls mit Knochenmaterial aufgefüllt werden, um nach Einsetzen des Implantates einen im wesentlichen voll ausgefüllten Zwischenwirbelraum zu ergeben. Für eine Verbindung der Knochenmasse außerhalb des Implantates im Zwischenwirbelraum ist die mindestens eine Vertiefung in der zum Inneren des Wirbelkörpers gewandten Begrenzungsfläche von großem Vorteil.

Gemäß einer bevorzugten Ausführungsform ist die Dicke des Implantates im mittleren Abschnitt durch die Vertiefung geringer als in den daran anschliessenden, außen liegenden Abschnitten. Dadurch ist das Implantat bei engen Platzverhältnissen leichter in den Zwischenwirbelraum einführbar, und ein Verwachsen mit umliegendem Knochenmaterial wird begünstigt. Das Implantat kann aufgrund der Vertiefung beim Einführen an der Nervenwurzel ohne Verlagerung derselben vorbeigeführt werden. Darüber hinaus kann ein im nicht vom Implantat bedeckten Zwischenwirbelraum eingesetzter Knochenspan mit bogenförmiger bzw. konvexer Oberfläche bzw. Außenkontur über eine größere Umfangsfläche an eine derartige Vertiefung in Anlage gebracht werden, wodurch ein nachträgliches Verwachsen begünstigt wird.

Um Verletzungen der das Implantat umgebenden Bereiche der Wirbelsäule zu vermeiden und einen im wesentlichen formschlüssigen Abschluß des Implantates mit den Außenkonturen der Wirbelkörper nach Einbau des Implantates zu erzielen, ist vorgesehen, daß die zu den Bogenwurzeln gewandten Außenflächen des Implantates abgerundet, insbesondere der Außenkontur des Wirbelkörpers im Bereich der Bogenwurzeln im wesentlichen folgend, ausgebildet sind, wie dies einer weiteren bevorzugten Ausführungsform des erfindungsgemäßen Implantates entspricht.

Für ein einfaches Ergreifen des Implantates beim Einsetzen durch entsprechende Instrumente sowie gegebenenfalls für ein Entfernen bzw. einen Ersatz desselben ist weiters bevorzugt vorgesehen, daß an den Außenflächen jeweils eine insbesondere mit einem Gewinde versehene und gegebenenfalls verschließbare Öffnung vorgesehen ist.

Neben dem Vorsehen von insbesondere mit einem Gewinde versehenen Öffnungen für ein Ergreifen oder Positionieren des erfindungsgemäßen Implantates ist gemäß einer weiters bevorzugten Ausführungsform vorgesehen, daß ausgehend von wenigstens einer Außenfläche und/oder der der konvex gekrümmten Begrenzungsfläche gegenüberliegenden Begrenzungsfläche wenigstens eine insbesondere schräg zur Längsachse des Implantates verlaufende Öffnung bzw. Bohrung vorgesehen ist, welche an der zum Inneren des Wirbelkörpers gewandten, konvex gekrümmten Begrenzungsfläche mündet. Durch wenigstens eine derartige, insbesondere schräg zur Längsachse des Implantates verlaufende Öffnung bzw. Bohrung, welche an der zum Inneren des Wirbelkörpers gewandten konvex gekrümmten Begrenzungsfläche mündet, wird es möglich, nach dem Einsetzen des Implantates durch Verwendung eines entsprechenden stab- bzw. rohrförmigen Werkzeuges bzw. Instrumentes im Bereich des Inneren des Wirbelkörpers liegenden Knochenmaterial nachträglich zu verdichten bzw. durch eine derartige Öffnung zusätzlich Knochenmaterial einzuführen, um eine entsprechend sichere Festlegung des Implantates zu erzielen und ein nachträgliches Verwachsen des Implantates, mit umliegendem Knochenmaterial zu unterstützen. Hierbei kann vorgesehen sein, daß im Bereich des nicht von dem Implantat abgedeckten Zwischenwirbelraumes entweder kleinstückiges Knochenmaterial angeordnet wird bzw. ein Knochenspan, beispielsweise aus dem Beckenkamm, eingesetzt wird, so daß durch die insbesondere schräg zur Längsachse des Implantates verlaufende Öffnung nachträglich ein entsprechendes Positionieren dieses Knochenspans oder ein Auffüllen von gegebenenfalls freibleibenden Zwischenräumen mit zusätzlichem Knochenmaterial bzw. ein ordnungsgemäßes Verdichten möglich wird. Hiebei kann weiters vorgesehen sein, daß die im Bereich der Außenflächen mündende Öffnung, welche ein Gewinde für ein zusammenwirken mit einem Instrument aufweist, unmittelbar durch die im Bereich der konvex gekrümmten Begrenzungsfläche mündende Öffnung fortgesetzt wird, so daß eine derartige durchgehende Öffnung bzw. ein derartiger Kanal zuerst unter Verwendung eines entsprechenden Werkzeuges zum Einsetzen des Implantates und in weiterer Folge unter Verwendung eines Instrumentes bzw. Werkzeuges mit einem gegenüber dem Gewindebereich verringerten Durchmesser zum Stopfen bzw. Verfüllen des Zwischenwirbelraumes herangezogen wird.

Um einen weiteren Bereich im Zwischenwirbelraum durch eine derartige, insbesondere schräg zur Längsachse das Implantates verlaufende Öffnung überstreichen zu können, ist gemäß einer weiters bevorzugten Ausführungsform vorgesehen, daß die Öffnung bzw. Bohrung zur konvex gekrümmten Begrenzungsfläche sich konisch erweiternd ausgebildet ist. Durch eine sich konisch erweiternde Öffnung kann somit ein mit entsprechend geringen Abmessungen ausgebildetes Werkzeug unter unterschiedlichen Neigungswinkeln in den Zwischenwirbelraum eingeführt werden.

Um eine Anpassung an die anatomisch vorgegebene Krümmung der Wirbelsäule zu ermöglichen sowie zur Aufrechterhaltung der Biomechanik der Wirbelsäule ist weiters bevorzugt vorgesehen, daß die an benachbarten Wirbelkörpern anliegenden Deckflächen des Implantates miteinander einen spitzen Winkel einschließen, wobei besonders bevorzugt vorgeschlagen wird, daß der von den Deckflächen miteinander eingeschlossene Winkel höchstens 25°, insbesondere höchstens 15° beträgt.

Zur Unterstützung der Verbindung des Implantates mit umgebendem Material der Wirbelkörper sowie für eine sichere Verankerung des Implantates ist das Implantat an den in Richtung benachbarter Wirbelkörper verlaufenden Begrenzungsflächen porös und/oder mit einer Profilierung, insbesondere einer Mehrzahl von Erhebungen und Vertiefungen, ausgebildet, wie dies einer weiteren bevorzugten Ausführungsform der Erfindung entspricht. Gemäß einer besonders bevorzugten Ausführungsform kann in diesem Zusammenhang vorgesehen sein, daß die poröse Oberfläche durch ein Sandstrahlen oder Vakuumbeschichten ausgebildet ist.

Um neben den für ein Auffüllen mit Knochenmasse erforderlichen Hohlräumen bzw. Durchbrechungen eine ausreichende Stützfunktion sowie Festigkeit des Implantates, welches eine vergleichsweise große Länge aufweist, da es in eingebautem Zustand im wesentlichen den gesamten Außenabstand der Bogenwurzeln überdeckt, sicherzustellen, ist erfindungsgemäß weiters bevorzugt vorgesehen, daß das Implantat zumindest in Richtung benachbarter Wirbelkörper zwischen den zum inneren des Wirbelkörpers und zum Dornfortsatz gewandten Begrenzungsflächen im wesentlichen hohl ausgebildet ist, wobei neben den die Begrenzungsflächen verbindenden Außenflächen wenigstens eine stegartige, in Richtung der Außenflächen verlaufende Verbindung zwischen den Begrenzungsflächen vorgesehen ist. Durch wenigstens eine derartige stegartige Verbindung wird eine entsprechende Erhöhung der Festigkeit des Implantates erzielt, wobei jedoch gleichzeitig ein ausreichend großer Freiraum im Implantat zur Ausfüllung mit Knochenmasse oder zum nachträglichen Einwachsen verbleibt.

Das erfindungsgemäße Implantat kann somit im wesentlichen solide bzw. vollflächig und in Richtung benachbarter Wirbelkörper verlaufend im wesentlichen frei von durchgehenden Durchbrechungen oder Ausnehmungen gebildet sein, wodurch sich insgesamt eine große Auflagefläche der benachbarten Wirbelkörper und damit eine bessere Abstützung derselben und insgesamt eine erhöhte Festigkeit des erfindungsgemäßen Implantates ergibt. Bei einer derartigen Ausbildung wird ein Anwachsen bzw. Einwachsen von umliegendem Knochenmaterial im wesentlichen an den Deckflächen und den in Richtung benachbarter Wirbelkörper verlaufenden Begrenzungsflächen und derart eine sichere Verankerung des Implantates im Zwischenwirbelraum erzielt. Für ein derartiges Anwachsen bzw. Umwachsen des umliegenden Knochenmaterials dienen im Bereich der Deckflächen des Implantates sowie der in Richtung benachbarter Wirbelkörper verlaufenden Begrenzungsflächen vorgesehene Profilierungen und/oder eine wenigstens teilweise poröse Oberfläche an diesen Begrenzungsflächen. Bei vorsehen wenigstens einer in Richtung benachbarter Wirbelkörper verlaufenden Durchbrechung, so daß das Innere des erfindungsgemäßen Implantates wenigstens teilweise hohl ausgebildet ist, läßt sich bei Einbringen von Knochenmaterial in diesen hohlen Bereich ein Verwachsen zwischen diesem im Hohlraum angeordneten Knochenmaterial und benachbarten Knochenflächen oder unmittelbar ein Durchwachsen von Knochenmaterial durch derartige Hohlräume erzielen.

Die Erfindung wird nachfolgend anhand von in der beiliegenden Zeichnung schematisch dargestellten Ausführungsbeispielen des erfindungsgemäßen Implantates näher erläutert. In dieser zeigen:
Fig. 1 eine schematische, teilweise Seitenansicht von zwei benachbarten Wirbelkörpern, zwischen welchen ein erfindungsgemäßes Implantat eingesetzt ist;
Fig. 2 schematische Draufsichten auf unterschiedliche Ausführungsformen eines Implantates; und
Fig. 3 schematische Draufsichten auf unterschiedliche Wirbelkörper mit darauf angeordneten Implantaten zur Veranschaulichung der Positionierung der Implantate in eingesetztem Zustand.

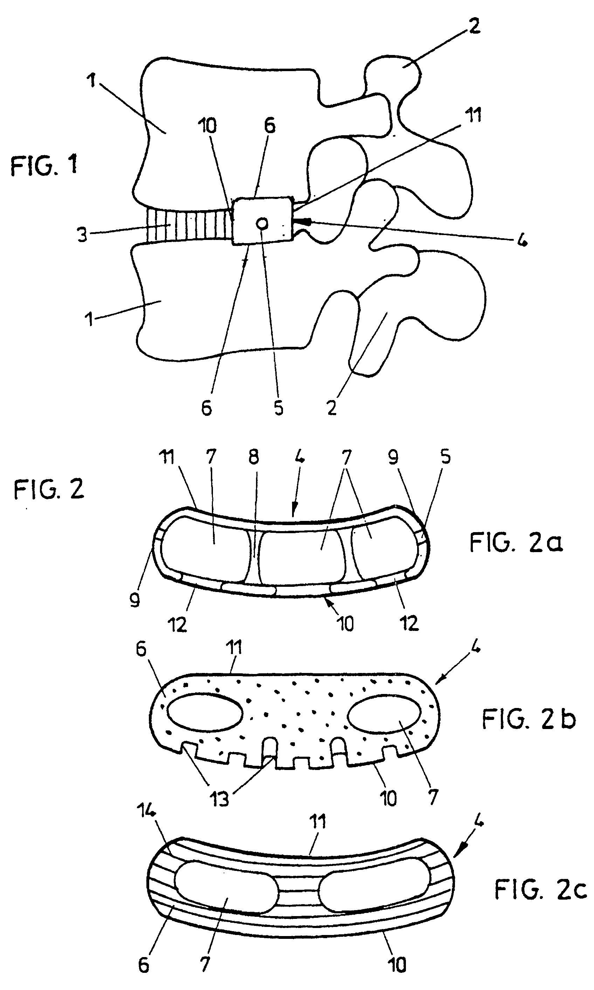

In Fig. 1 sind mit 1 schematisch zwei benachbarte Wirbelkörper und mit 2 jeweils Wirbelfortsätze der Wirbelkörper 1 bezeichnet.

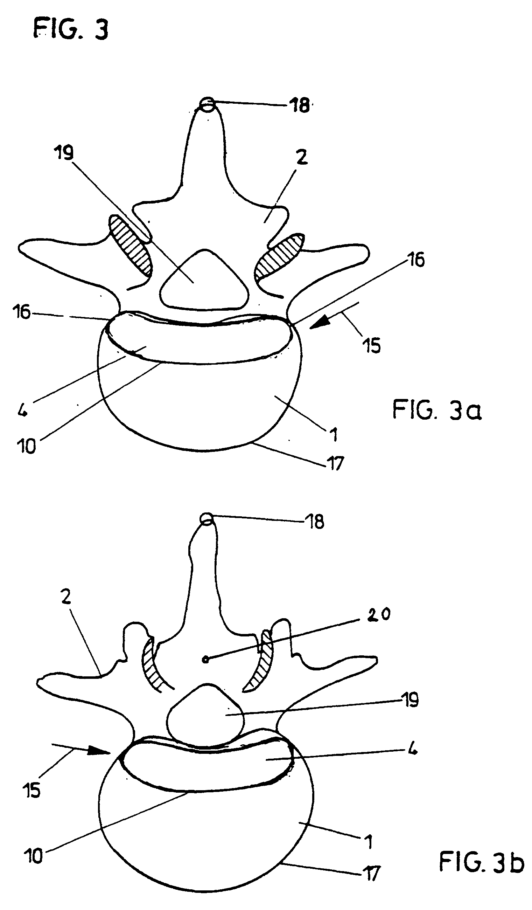

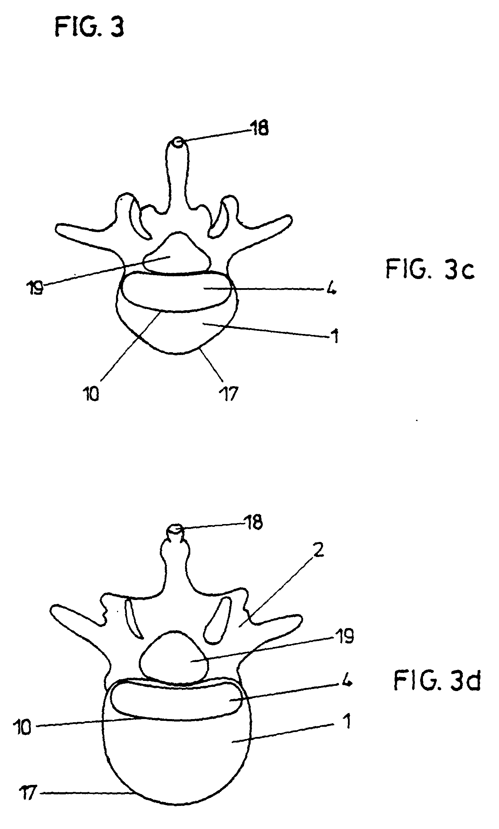

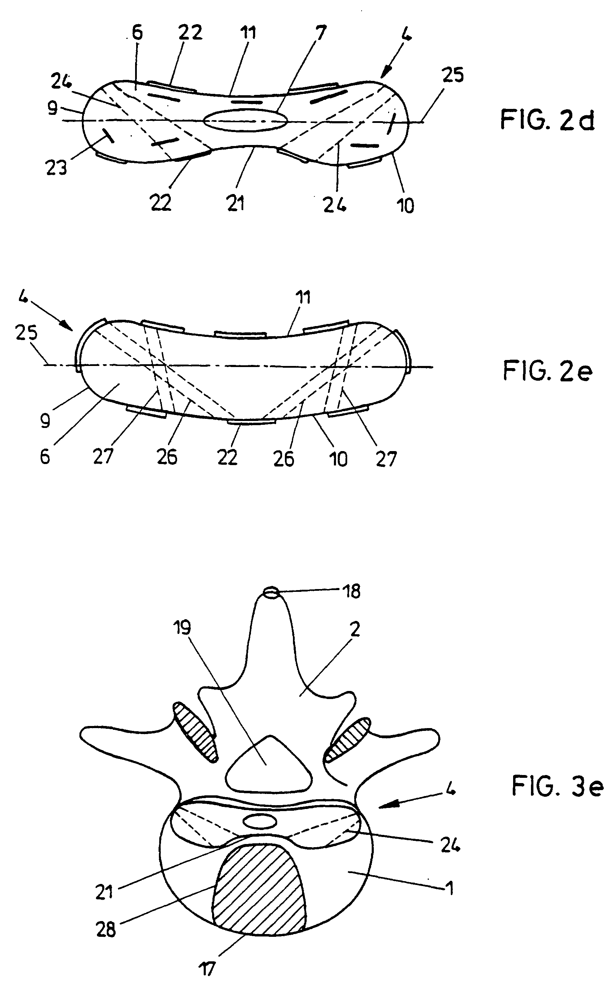

In dem Intervertebrairaum 3 ist nach einem Entfernen einer Bandscheibe in einem vorderen Bereich Knochenmasse vorgesehen, während in dem zum Dorn- bzw. Wirbelfortsatz 2 gewandten Bereich ein Implantat 4 eingesetzt ist, welches in Fig. 2 und 3 detaillierter dargestellt ist. Das Implantat 4 weist an seiner nach außen gewandten Außenfläche eine schematisch angedeutete Öffnung 5 auf, welche zum Ansetzen bzw. Festlegen eines Instrumentes beim Einsetzen des Implantates 4 dient und welche in eingebautem Zustand gegebenenfalls verschließbar ist.

Das Implantat 4 weist weiters an den Wirbelkörpern 1 anliegende Deckflächen 6 auf, welche in der in Fig. 1 gezeigten Ausführungsform miteinander einen spitzen Winkel einschließen, welcher beispielsweise 10° bis 15° beträgt. Durch diesen Winkel zwischen den Deckflächen 6 läßt sich eine Anpassung an die Krümmung der Wirbelsäule sowie an die biomechanischen Eigenschaften der Wirbelsäule erzielen, wobei bei den in Fig. 3 dargestellten unterschiedlichen Wirbelkörpern 1 neben der Wahl unterschiedlich großer Implantate 4 zusätzlich durch Wahl der Winkel zwischen den Deckflächen 6 der unterschiedlichen Krümmung der Wirbelsäule in verschiedenen Teilbereichen entsprechend Rechnung getragen werden kann.

Weiters ist aus Fig. 1 und in größerem Detail aus Fig. 3 ersichtlich, daß das Implantat 4 in eingesetztem Zustand jeweils nur im zu den Wirbelfortsätzen 2 gewandten Teilbereich der Wirbelkörper 1 angeordnet ist, wobei in diesem rückwärtigen Teil die größten Kräfte und Belastungen wirksam werden und somit durch das Implantat 4 eine wirkungsvolle Abstützung der Wirbelkörper 1 und Aufnahme der Belastungen möglich wird.

In Fig. 2 sind unterschiedliche Ausführungen des Implantates 4 jeweils in einer Draufsicht in größerem Detail dargestellt. Bei der Ausbildung gemäß Fig. 2a ist ersichtlich, daß das Implantat 4 drei, im wesentlichen senkrecht verlaufende, durchgehende Ausnehmungen bzw. Durchbrechungen 7 aufweist, welche vor dem Einbau des Implantates 4 mit Knochenmasse aufgefüllt werden, so daß nach dem Einsetzen des Implantates 4 zwischen Wirbelkörpern 1 ein Verwachsen mit den unmittelbar anschließenden Wirbelkörpern 1 erfolgen kann. Die Durchbrechungen 7 sind in Längsrichtung des Implantates 4 durch stegartige Verbindungen 8 sowie die Außenflächen 9 des Implantates 4 begrenzt, so daß sich eine entsprechend widerstandsfähige und eine hohe Festigkeit aufweisende Konstruktion erzielen läßt. Die Begrenzungsflächen des Implantates 4 in Richtung zum Inneren der Wirbelkörper 1 sowie in Richtung zu den Wirbelfortsätzen 2 sind mit 10 und 11 bezeichnet.

In Fig. 2a sind die an den Außenflächen 9 vorgesehenen Öffnungen zum Ergreifen des Implantates 4 mit nicht näher dargestellten Instrumenten wiederum mit 5 angedeutet und es sind weiters schematisch angedeutete Öffnungen bzw. Durchbrechungen 12 in der zum Inneren der Wirbelkörper 1 gewandten, konvex gekrümmten Begrenzungsfläche 10 des Implantates ersichtlich. Neben einem Verbinden mit umliegender Knochenmasse durch die Durchbrechungen 7 kann auch über die Öffnungen 12 eine Verbindung zwischen dem im Inneren der Durchbrechungen 7 eingebrachten Knochenmaterial mit umliegendem Knochenmaterial 3 des Zwischenwirbelraumes erfolgen. Die zum Dornfortsatz gewandte Begrenzungsfläche 11 des Implantates 4 weist demgegenüber keine Öffnungen oder Durchbrechungen auf, um ein Austreten von Knochenmaterial an dieser stelle und ein gegebenenfalls mögliches Eindringen desselben in den Bereich des anschließenden Wirbelkanals zu vermeiden, wie dies deutlicher aus Fig. 3 ersichtlich ist.

In Fig. 2a ist weiters erkennbar, daß das Implantat 4, in Draufsicht gesehen, eine im wesentlichen bogen- bzw. sichelförmige Außenkontur aufweist, welche ein leichtes und sicheres, laterales Einbringen von hinten in den Zwischenwirbelraum ermöglicht, wie dies ebenfalls aus Fig. 3 deutlich ersichtlich ist.

Bei der Ausbildung gemäß Fig. 2b sind wiederum im wesentlichen senkrechte Durchbrechungen 7 zur Aufnahme von Knochenmaterial vorgesehen. Anstelle oder gegebenenfalls zusätzlich zu den Ausnehmungen 12 in der gekrümmten Begrenzungsfläche 10 sind bei dieser Ausbildung abgesetzte bzw. hinterschnittene, im wesentlichen senkrecht bzw. in Richtung benachbarter Wirbelkörper verlaufende Teilbereiche bzw. Durchbrechungen 13 vorgesehen, wobei durch Eindringen von Knochenmaterial in diese ausgeschnittenen Bereiche bzw. Vertiefungen 13 ebenfalls eine sichere Verankerung des Implantates 4 ermöglicht wird. Zur Unterstützung der Verankerung kann bei dieser Ausbildung zusätzlich vorgesehen sein, daß zumindest die Begrenzungsfläche 10 sowie die Deckflächen 6 des Implantates 4 entsprechend aufgerauht oder porös ausgebildet sind, um eine Verbindung mit umliegendem Knochenmaterial zu erleichtern. Bei der Ausbildung gemäß Fig. 2b ist ersichtlich, daß neben der wiederum konvex gekrümmten Begrenzungsfläche 10, welche das laterale Einbringen des Implantates 4 unterstützt, die Begrenzungsfläche 11 im wesentlichen gerade bzw. eben ausgebildet ist, wodurch gegebenenfalls die Herstellung des Implantates 4 vereinfacht wird.

Bei der Ausbildung gemäß Fig. 2c sind an den Deckflächen 6 im wesentlichen entsprechend der Krümmung der Begrenzungsflächen 10 und 11 verlaufende Profilierungen 14 in Form von Erhebungen und Vertiefungen vorgesehen, welche eine zusätzliche Verankerung des Implantates 4 ermöglichen.

Bei der Darstellung gemäß Fig. 2d ist an der konvex gekrümmten Begrenzungsfläche 10) in Abwandlung der Ausführungsform gemäß Fig. 2b lediglich eine Vertiefung bzw. Ausnehmung 21 vorgesehen, so daß sich im im mittleren Abschnitt des Implantates 4 eine gegenüber den Wandbereichen geringere Dicke ergibt, wodurch ein verbessertes Einbringen des Implantates 4 im Bereich der Nervenwurzel, wie dies unter Berücksichtigung der nachfolgenden Fig. 3 noch deutlicher ersichtlich werden wird und damit ein geringeres Verletzungsrisiko erzielbar ist. Darüberhinaus ist an den Begrenzungsflächen 10 und 11 vorgesehen, daß vorragend von diesen schematisch mit 22 bezeichnete Profilierungen bzw. Erhebungen ausgebildet sind, welche ein leichteres verwachsen mit umliegendem Knochenmaterial ermöglichen. Ähnliche Profilierungen bzw. Erhebungen oder komplementär ausgebildete Vertiefungen sind auch an den Deckflächen 6 vorgesehen und jeweils mit 23 bezeichnet, wobei in Abwandlung von der Ausführungsform gemäß Fig. 2c diese Profilierungen jeweils eine im Vergleich zur Gesamterstreckung der Deckfläche 6 verringerte Ausdehnung aufweisen, während bei der Ausbildung gemäß Fig. 2c die Profilierungen im wesentlichen durchgehend über die gesamte Deckfläche 6 verlaufend ausgebildet sind. Anstatt derartiger Profilierungen bzw. Erhebungen 22, 23 kann an den Deckflächen bzw. den Begrenzungsflächen 9, 10 und 11 ähnlich wie bei der Ausführungsform gemäß Fig. 2b eine entsprechend rauhe bzw. poröse Oberfläche vorgesehen sein, welche beispielsweise durch ein Sandstrahlen oder Vakuumbeschichten hergestellt wird.

Ausgehend von den jeweils außen liegenden Begrenzungsflächen 9 sind bei der Ausbildung gemäß Fig. 2d im wesentlichen auf halber Höhe des Implantates 4 verlaufende, strichliert angedeutete Öffnungen bzw. Bohrungen 24 vorgesehen, welche an der konvex gekrümmten Begrenzungsfläche 10 münden. Wie aus der Darstellung gemäß Fig. 2d ersichtlich, sind diese Öffnungen bzw. Bohrungen 24 in Richtung zur Begrenzungsfläche 10 sich konisch erweiternd ausgebildet. Die im Bereich der Außenflächen 9 vorgesehenen Eintrittsöffnungen können beispielsweise unmittelbar ähnlich wie die Durchbrechung 5 mit einem Gewinde versehen sein, um ein Ergreifen mit einem Instrument beim Einführen des Implantates 4 zu ermöglichen. Nach erfolgtem Einsetzen kann unter Verwendung eines einen geringen Durchmesser aufweisenden Instrumentes bzw. Stopfwerkzeuges dieses durch die durchgehende Öffnung 24 in den Bereich vor der konvexen Oberfläche 10 bzw. den Bereich der Vertiefung 21 eingeführt werden, um an dieser Stelle eine Verdichtung von beispielsweise bereits im Zwischenwirbelraum befindlicher Knochenmasse zu ermöglichen. Gegebenenfalls kann durch diese Öffnungen 24 zusätzliches Knochenmaterial in den vor der konvexen Begrenzungsfläche 10 liegenden Bereich eingebracht und verdichtet werden. Hiebei aus Fig. 2d weiters ersichtlich, daß die Öffnung bzw. Bohrung 24 schräg zur schematisch mit 25 angedeuteten Längsachse des Implantates 4 verläuft.

Bei der abgewandelten Ausführungsform gemäß Fig. 2e sind an den äußeren Begrenzungsflächen 9 sowie an der konkaven und der konvexen Begrenzungsfläche 10 und 11 wiederum mit 22 bezeichnete Profilierungen bzw. Erhebungen angedeutet, wobei ähnliche Profilierungen auch an den jeweiligen Deckflächen 6 vorgesehen sein können. Während bei der Ausbildung gemäß Fig. 2d die durchgehende Bohrung bzw. Öffnung 24 sich konisch erweiternd ausgebildet ist, um einen großen Bereich mit einem Stopfwerkzeug überstreichen zu können, sind bei der Ausführungsform gemäß Fig. 2e einander kreuzende Bohrungen 26 und 27 vorgesehen, welche wiederum geneigt zur Längsachse 25 des Implantates 4 verlaufen und durch Verwendung von entsprechenden Stopfwerkzeugen wiederum ein Verfestigen von Knochenmasse, welche vor der konvexen Begrenzungsfläche 10 liegt, ermöglichen. Insbesondere das im Bereich der Außenfläche 9 mündende Ende der Bohrungen 26 kann für das Einsetzen des Implantates 4 wiederum mit einem Werkzeug zusammenwirken. weiters ist aus Fig. 2e ersichtlich, daß neben einer porösen Oberfläche bzw. entsprechenden Profilierungen an den Deckflächen 6 keine sich insbesondere vollständig durch das Implantat 4 erstreckende Durchbrechungen vorgesehen sind, so daß hiedurch eine entsprechend massive und eine hohe Festigkeit aufweisende Ausbildung eines Implantates 4 erzielbar ist.

In Fig. 3 sind unterschiedliche Wirbel mit daran schematisch angeordneten Implantaten 4 mit an die Wirbel jeweils angepaßter Größe dargestellt. Die Implantate 4 können aufgrund ihrer zumindest an der zum Inneren der Wirbelkörper 1 konvex gekrümmten Begrenzungsfläche 10 seitlich von hinten im Sinne der Pfeile 15 eingebracht werden, ohne daß eine Entfernung von Band- und Knochenmaterial im Bereich der Wirbelfortsätze 2 notwendig wäre. weiters ist aus Fig. 3 ersichtlich, daß die Implantate 4 eine Breite aufweisen, so daß die Implantate 4 jeweils nur den zu den Wirbelfortsätzen 2 gewandten, rückwärtigen Bereich, und insbesondere maximal die Hälfte des Wirbelkörpers 1, wie dies in Fig. 3c angedeutet ist, oder etwa ein Drittel der Fläche der Wirbelkörper 1 überdecken, während die Implantate 4 eine Länge aufweisen, welche im wesentlichen der gesamten Erstreckung der Wirbelkörper 1 in diesem Bereich, insbesondere der Länge des Außenabstandes der Bogenwurzeln 16, entspricht.

Darüber hinaus weist die Begrenzungsfläche 10 einen Krümmungsradius auf, welcher im Bereich von 30 bis 90 %, insbesondere 40 bis 80 %, des Abstandes zwischen der Vorderkante 17 des Wirbelkörpers 1 und der Spitze 18 des Dornfortsatzes 2 beträgt, wobei der Mittelpunkt des die Begrenzungsfläche 10 definierenden Kreisbogens bei der Ausbildung gemäß den Fig. 3a, 3c und 3d in der Spitze 18 des Dornfortsatzes 2 gewählt wird. Demgegenüber ist bei der Ausbildung gemäß Fig. 3b der Mittelpunkt des Kreisbogens in der Basis des Dornfortsatzes 2 gewählt und mit 20 bezeichnet, wodurch sich insgesamt durch die stärkere Krümmung der in Fig. 3 dargestellten Ausführungsform des Implantates 4 die Einführung desselben entsprechend Pfeil 15 weiter vereinfachen läßt.

Bei einer bogenförmigen Ausbildung des Implantates 4, wie sie beispielsweise in den Fig. 2a und 2c bis 2e detailliert dargestellt ist, kann weiters der Krümmungsradius der zum Dornfortsatz 2 gewandten Begrenzungsfläche 11 des Implantates 4 zwischen 10 und 80 %, insbesondere 20 bis 60 %, des Abstandes zwischen der Vorderkante 17 des Wirbelkörpers 1 und der Spitze 18 des Dornfortsatzes 2 betragen, wobei als Drehmittelpunkte wiederum die Positionen 18 bzw. 20 gewählt werden.

Der Wirbelkanal, welcher insbesondere geschützt werden maß, ist in Fig. 3 jeweils mit 19 bezeichnet.

Bei der Darstellung in Fig. 3e ist angedeutet, daß in dem nicht von einem Implantat 4 überdeckten Zwischenwirbelraum ein schematisch mit 28 bezeichneter Knochenspan, welcher beispielsweise dem Beckenkamm entnommen wurde, eingesetzt ist. Nach Einbringen des Implantates 4 zwischen benachbarte Wirbelkörper 1 läßt sich bei der bei der schematisch angedeuteten Ausführungsform eines Implantates 4, welches beispielsweise dem Implantat gemäß Fig. 2d entspricht, durch die wiederum mit 24 angedeuteten Öffnungen ein nachträgliches Verfüllen des Zwischenwirbelraumes bzw. verdichten von Material auch im Bereich des eingesetzten, getrennten Knochenspans 28 erzielen. Weiters in Fig. 3e angedeutet, daß bei Vorsehen einer Vertiefung 21 derart nicht nur ein erleichtertes Einbringen des Implantates 4 unter Verringerung des Verletzungsrisikos im Bereich der Nervenwurzel erzielbar ist, sondern daß auch ein entsprechend großer Knochenspan 28 eingesetzt werden kann, welcher durch die durch die Vertiefung 21 vorgesehene Vergrößerung der Oberfläche bei zusätzlichem Vorsehen von entsprechenden Profilierungen, welche der Einfachheit halber in Fig. 3e nicht dargestellt sind, eine entsprechend sichere Verankerung der Implantates 4 und Verbindung mit umliegendem Material erzielen läßt.

Selbstverständlich können anstelle der in Fig. 3 jeweils schematisch angedeuteten Implantate 4 beliebige Implantate, welche in anderen Figuren gezeigt sind, verwendet werden, um gewünschte Effekte bzw. eine Anpassung an entsprechende Erfordernisse zu ermöglichen. weiters können naturgemäß Einzelmerkmale der insbesondere in den Fig. 2a bis 2e dargestellten unterschiedlichen Ausführungsformen von Implantaten 4 abweichend von den gezeigten Ausbildungen zur Erzielung gewünschter Eigenschaften eines Implantates 4 entsprechend kombiniert werden.

In der Zeichnung und in der Beschreibung der bevorzugten Ausführungsbeispiele sind auch Implantate vertreten, die keine Vertiefung in ihrer zum Inneren des Wirbelkörpers gewandten Begrenzungsfläche 10 aufweisen. Insofern dienen diese Ausführungsbeispiele lediglich der Erläuterung des Erfindungsgegenstandes, bei dem in jedem Fall mindestens eine derartige Vertiefung vorgesehen ist.

## Patentansprüche

1. Implantat zum Einsetzen zwischen Wirbelkörper (1) der Wirbelsäule, wobei das im Querschnitt im wesentlichen rechteckige oder trapezoidförmige Implantat (4) an zu benachbarten Wirbelkörpern (1) gewandten Deckflächen (6) porös und/oder mit einer Profilierung (14) ausgebildet ist, mit einer Breite, welche maximal die zum Dornfortsatz (2) gewandte, rückwärtige Hälfte des Wirbelkörpers (1) überdeckt, und mit einer den rückwärtigen Bereich des Wirbelkörpers (1) im wesentlichen übergreifende Länge, wobei das Implantat (4) an seiner zum Inneren des Wirbelkörpers (1) gewandten, in Richtung benachbarter Wirbelkörper (1) verlaufenden Begrenzungsfläche (10) konvex gekrümmt ist und an seiner zum Dornfortsatz (2) gewandten Begrenzungsfläche (11) konkav gekrümmt ausgebildet ist, so daß sich eine sichelförmige Ausgestaltung ergibt, **dadurch gekennzeichnet, daß** die zum Innern des Wirbelkörpers (1) gewandte Begrenzungsfläche (10) wenigstens eine Vertiefung (13, 21) aufweist.

2. Implantat nach Anspruch 1, **dadurch gekennzeichnet, daß** die zum Dornfortsatz (2) gewandte Begrenzungsfläche (11) frei von Durchbrechungen ausgebildet ist.

3. Implantat nach einem der Ansprüche 1 oder 2, **dadurch gekennzeichnet, daß** die zu den Bogenwurzeln (16) gewandten Außenflächen (9) des Implantates (4) abgerundet ausgebildet sind.

4. Implantat nach einem der voranstehenden Ansprüche, **dadurch gekennzeichnet, daß** an den zu den Bogenwurzeln (16) gewandten Außenflächen (9) jeweils eine Öffnung (5, 24, 26) vorgesehen ist.

5. Implantat nach Anspruch 4, **dadurch gekennzeichnet, daß** die Öffnung (5, 24, 25) mit einem Gewinde versehen ist.

6. Implantat nach einem der Ansprüche 4 oder 5, **dadurch gekennzeichnet, daß** die Öffnung (5, 24, 25) verschließbar ist.

7. Implantat nach einem der voranstehenden Ansprüche, **dadurch gekennzeichnet, daß** ausgehend von wenigstens einer Außenfläche (9) wenigstens eine Öffnung (24, 26, 27) vorgesehen ist, welche an der zum Inneren des Wirbelkörpers gewandten, konvex gekrümmten Begrenzungsfläche (10) mündet.

8. Implantat nach Anspruch 7, **dadurch gekennzeichnet, daß** die Öffnung (24, 26, 27) schräg zur Längsachse (25) des Implantates (4) verläuft.

9. Implantat nach Anspruch 7 oder 8, **dadurch gekennzeichnet, daß** die Öffnung (24) zur konvex gekrümmten Begrenzungsfläche (10) sich konisch erweiternd ausgebildet ist.

10. Implantat nach einem der voranstehenden Ansprüche, **dadurch gekennzeichnet, daß** die an benachbarten Wirbelkörpern (1) anliegenden Deckflächen (6) des Implantates (4) miteinander einen spitzen Winkel einschließen.

11. Implantat nach Anspruch 10, **dadurch gekennzeichnet, daß** der von den Deckflächen (6) miteinander eingeschlossene Winkel höchstens 25°, insbesondere höchstens 15° beträgt.

12. Implantat nach einem der Ansprüche 1 bis 11, **dadurch gekennzeichnet, daß** das Implantat (4) an den in Richtung benachbarter Wirbelkörper (1) verlaufenden Begrenzungsflächen (9, 10, 11) porös und/oder mit einer Profilierung (14, 22, 23) ausgebildet ist.

13. Implantat nach Anspruch 12, **dadurch gekennzeichnet, daß** die Profilierung (14, 22, 23) durch eine Mehrzahl von Erhebungen und Vertiefungen gebildet wird.

14. Implantat nach Anspruch 12, **dadurch gekennzeichnet, daß** die poröse Oberfläche durch ein Sandstrahlen oder Vakuumbeschichten ausgebildet ist.

15. Implantat nach einem der voranstehenden Ansprüche, **dadurch gekennzeichnet, daß** das Implantat (4) zumindest in Richtung benachbarter Wirbelkörper (1) zwischen den zum Inneren des Wirbelkörpers (1) und zum Dornfortsatz (2) gewandten Begrenzungsflächen (10, 11) im wesentlichen hohl ausgebildet ist, wobei neben den die Begrenzungsflächen (10, 11) verbindenden Außenflächen (9) wenigstens eine stegartige, in Richtung der Außenflächen verlaufende Verbindung (8) zwischen den Begrenzungsflächen (10, 11) vorgesehen ist.

16. Implantat nach einem der voranstehenden Ansprüche, **dadurch gekennzeichnet, daß** sich die Vertiefung in Richtung benachbarter Wirbelkörper (1) erstreckt.

17. Implantat nach einem der voranstehenden Ansprüche, **dadurch gekennzeichnet, daß** sich die Vertiefung (13, 21) im mittleren Bereich des Implantates befindet.

18. Implantat nach einem der voranstehenden Ansprüche, **dadurch gekennzeichnet, daß** die Dicke des Implantates (4) im mittleren Abschnitt durch die Vertiefung (21) geringer ist als in den daran anschließenden, außen liegenden Abschnitten.

## Claims

1. An implant for insertion between vertebral bodies (1) of the vertebral column, the implant (4), which is substantially rectangular or trapezoidal in cross-section, being designed so as to be porous and/or with profiling (14) on covering surfaces (6) facing adjacent vertebral bodies (1), with a width which covers, at the most, the rear half of the vertebral body (1) facing the spinal process (2), and with a length substantially extending over the rear region of the vertebral body (1), wherein the implant (4) is curved convexly on its boundary surface (10) facing the interior of the vertebral body (1) and extending in the direction of adjacent vertebral bodies (1), and is designed so as to be curved concavely on its boundary surface (11) facing the spinal process (2), resulting in a crescent-shaped structure, **characterised in that** the boundary surface (10) facing the interior of the vertebral body (1) has at least one depression (13, 21).

2. An implant according to Claim 1, **characterised in that** the boundary surface (11) facing the spinal process (2) is designed so as to be free of through-holes.

3. An implant according to one of Claims 1 or 2, **characterised in that** the exterior surfaces (9) of the implant (4) facing the curve roots (16) are designed so as to be rounded.

4. An implant according to one of the preceding claims, **characterised in that** an opening (5, 24, 26) is provided in each case on the exterior surfaces (9) facing the curve roots (16).

5. An implant according to Claim 4, **characterised in that** the opening (5, 24, 25) is provided with a thread.

6. An implant according to one of Claims 4 or 5, **characterised in that** the opening (5, 24, 25) is closable.

7. An implant according to one of the preceding claims, **characterised in that** at least one opening (24, 26, 27) is provided, proceeding from at least one exterior surface (9), which opens on the convexly curved boundary surface (10) facing the interior of the vertebral body.

8. An implant according to Claim 7, **characterised in that** the opening (24, 26, 27) extends obliquely to the longitudinal axis (25) of the implant (4).

9. An implant according to Claim 7 or 8, **characterised in that** the opening (24) is designed so as to widen in a conical shape to the convexly curved boundary surface (10).

10. An implant according to one of the preceding claims, **characterised in that** the covering surfaces (6) of the implant (4) abutting adjacent vertebral bodies (1) form an acute angle with one another.

11. An implant according to Claim 10, **characterised in that** the angle formed by the covering surfaces (6) with one another is at most 25°, more particularly at most 15°.

12. An implant according to one of Claims 1 to 11, **characterised in that** the implant (4) is designed so as to be porous and/or with profiling (14, 22, 23) on the boundary surfaces (9, 10, 11) extending in the direction of adjacent vertebral bodies (1).

13. An implant according to Claim 12, **characterised in that** the profiling (14, 22, 23) is formed by a plurality of projections and depressions.

14. An implant according to Claim 12, **characterised in that** the porous surface is created by means of sandblasting or vacuum coating.

15. An implant according to one of the preceding claims, **characterised in that** the implant (4) is designed so as to be substantially hollow, at least in the direction of adjacent vertebral bodies (1), between the boundary surfaces (10, 11) facing the interior of the vertebral body (1) and the spinal process (2), wherein there is provided, as well as the exterior surfaces (9) connecting the boundary surfaces (10, 11), at least one web-like connection (8) between the boundary surfaces (10, 11), extending in the direction of the exterior surfaces.

16. An implant according to one of the preceding claims, **characterised in that** the depression extends in the direction of adjacent vertebral bodies (1).

17. An implant according to one of the preceding claims, **characterised in that** the depression (13, 21) is located in the central region of the implant.

18. An implant according to one of the preceding claims, **characterised in that** the thickness of the implant (4) is less in the central portion as a result of the depression (21) than in the externally located portions adjoining the said depression.

## Revendications

1. Implant à insérer entre des corps vertébraux (1) de la colonne vertébrale, l'implant (4) de section transversale en forme sensiblement de rectangle ou de trapèze étant réalisé poreux et/ou avec profilage (14) sur des surfaces de recouvrement (6) tournées vers des corps vertébraux (1) voisins, avec une largeur qui recouvre au maximum la moitié postérieure du corps vertébral (1) qui est tournée vers l'apophyse épineuse (2), et avec une longueur chevauchant sensiblement la région postérieure du corps vertébral (1), l'implant (4) étant incurvé de manière convexe sur sa surface de limitation (10). tournée vers l'intérieur du corps vertébral (1) et s'étendant en direction du corps vertébral (1) voisin et incurvé de manière concave sur sa surface de limitation (11) tournée vers l'apophyse épineuse (2), de sorte que l'on obtient une configuration en forme de croissant, **caractérisé en ce que** la surface de limitation (10) tournée vers l'intérieur du corps vertébral (1) présente au moins un creux (13, 21).

2. Implant selon la revendication 1, **caractérisé en ce que** la surface de limitation (11) tournée vers l'apophyse épineuse (2) est réalisée exempte de traversées.

3. Implant selon l'une ou l'autre des revendications 1 et 2, **caractérisé en ce que** les surfaces extérieures (9) de l'implant (4), tournées vers les pédicules vertébraux (16), sont réalisées arrondies.

4. Implant selon l'une des revendications précédentes, **caractérisé en ce qu'**il est prévu une ouverture (5, 24, 26) sur chacune des surfaces extérieures (9) tournées vers les pédicules vertébraux.

5. Implant selon la revendication 4, **caractérisé en ce que** l'ouverture (5, 24, 25) est pourvue d'un pas de vis.

6. Implant selon l'une ou l'autre des revendications 4 et 5, **caractérisé en ce que** l'ouverture (5, 24, 25) peut être fermée.

7. Implant selon l'une des revendications précédentes, **caractérisé en ce qu'**en partant d'au moins une surface extérieure (9), il est prévu au moins une ouverture (24, 26, 27) qui débouche sur la surface de limitation (10) incurvée de manière convexe et tournée vers l'intérieur du corps vertébral.

8. Implant selon la revendication 7, **caractérisé en ce que** l'ouverture (24, 26, 27) s'étend en oblique vers l'axe longitudinal (25) de l'implant (4).

9. Implant selon l'une ou l'autre des revendications 7 et 8, **caractérisé en ce que** l'ouverture (24) est réalisée en s'élargissant en forme de cône par rapport à la surface de limitation (10) incurvée de manière convexe.

10. Implant selon l'une des revendications précédentes, **caractérisé en ce que** les surfaces de recouvrement (6) de l'implant (4) en contact avec des corps vertébraux voisins (1) forment ensemble un angle aigu.

11. Implant selon la revendication 10, **caractérisé en ce que** l'angle formé par les surfaces de recouvrement (6) est au plus de 25°, en particulier au plus de 15°.

12. Implant selon l'une des revendications 1 à 11, **caractérisé en ce que** l'implant (4) est réalisé poreux et/ou avec un profilage (14, 22, 23) sur les surfaces de limitation (9, 10, 11) s'étendant en direction de corps vertébraux (1) voisins.

13. Implant selon la revendication 12, **caractérisé en ce que** le profilage (14, 22, 23) est formé par une pluralité de bosses et de creux.

14. Implant selon la revendication 12, **caractérisé en ce que** la surface poreuse est réalisée par sablage ou par enduction sous vide.

15. Implant selon l'une des revendications précédentes, **caractérisé en ce que** l'implant (4) est réalisé sensiblement creux au moins en direction de corps vertébraux (1) voisins entre les surfaces de limitation (10, 11) tournées vers l'intérieur du corps vertébral (1) et vers l'apophyse épineuse (2), et à côté des surfaces extérieures (9) reliant les surfaces de limitation (10, 11), il est prévu entre les surfaces de limitation (10, 11) au moins une liaison (8) en forme de traverse qui s'étend en direction des surfaces extérieures.

16. Implant selon l'une des revendications précédentes, **caractérisé en ce que** le creux s'étend en direction de corps vertébraux (1) voisins.

17. Implant selon l'une des revendications précédentes, **caractérisé en ce que** le creux (13, 21) se trouve dans la région médiane de l'implant.

18. Implant selon l'une des revendications précédentes, **caractérisé en ce que** l'épaisseur de l'implant (4) dans le tronçon médian est, en raison du creux (21), plus faible que dans les tronçons adjacents situés à l'extérieur.
